# EUROPÄISCHE PATENTSCHRIFT

(11) **EP 1 285 630 B1**
(45) Veröffentlichungstag und Bekanntmachung des Hinweises auf die Patenterteilung: **07.08.2013**
(21) Anmeldenummer: 01119822.3
(22) Anmeldetag: 16.08.2001
(51) Int. Cl.: A61B 17/17

(54) **Chirurgischer Instrumentensatz**
Surgical instrument set
Jeu d'instruments chirurgicaux

(43) Veröffentlichungstag der Anmeldung: 26.02.2003
(73) Patentinhaber: WALDEMAR LINK GmbH & Co. KG, 22339 Hamburg (DE)
(72) Erfinder: Thietje, Roland Dr. BG Unfallkrankenhaus Hamburg, 21033 Hamburg (DE); Nürnberg, Hans-J. BG Unfallkrankenhaus Hamburg, 21033 Hamburg (DE)
(74) Vertreter: Glawe, Delfs, Moll

(56) Entgegenhaltungen:
- EP-A- 1 086 655
- WO-A-01/28439
- DE-A- 3 800 482
- US-A- 3 867 932
- US-A- 4 798 213
- US-A- 6 083 225

## Beschreibung

Die Erfindung wird in Anspruch 1 definiert. US 6,083,225 lehrt einen chirurgischen Instrumentensatz gemäß dessen Oberbegriff.

Die Erfindung verzichtet darauf, dem Arzt innerhalb der Gewebeschutzhülse Spielraum für das örtliche Ansetzen des Werkzeugs zu belassen. Statt dessen sind die Gewebeschutzhülse und das Werkzeug so aufeinander abgestimmt, daß das Werkzeug eindeutig durch die Gewebeschutzhülse geführt wird, indem die Mittellinie der Gewebeschutzhülse und des Werkzeugs zusammenfallen. Wenn sie richtig positioniert ist, ist auch das Werkzeug richtig positioniert. Der Arzt braucht sich daher lediglich auf die Positionierung der Gewebeschutzhülse bzw. des zum Einführen der Gewebeschutzhülse verwendeten Trokars zu konzentrieren. Die Operation erfolgt zweckmäßigerweise unter Röntgenkontrolle.

Zweckmäßigerweise ist der Führungsabschnitt des Werkzeugs mit einer Längenmeßskala versehen. Durch Vergleich dieser Skala mit dem hinteren Ende der Gewebeschutzhülse kann er auf diese Weise jederzeit feststellen, welche Stellung das vordere, nicht unmittelbar sichtbare Ende des Werkzeugs im Verhältnis zur Gewebeschutzhülse hat. Wenn das vordere Ende der Gewebeschutzhülse auf die Knochenoberfläche aufgesetzt ist, wie es normalerweise der Fall ist, erhält er auf diese Weise auch Aufschluß über die Lage des Werkzeugs im Verhältnis zur Knochenoberfläche.

Zweckmäßigerweise korrespondiert die Nullmarkierung der Skala am Führungsabschnitt des Werkzeugs mit dem Ende der Gewebeschutzhülse, wenn die Spitze des Werkzeugs am vorderen Ende der Gewebeschutzhülse angekommen ist. Danach gibt die Skala darüber Aufschluß, wie weit das Werkzeug in bezug auf den Knochen vorgedrungen ist. Wenn das Werkzeug ein Lochbohrer oder Gewindebohrer ist, erfährt man auf diese Weise, wie tief die Bohrung ist. Wenn das Werkzeug ein Schraubendreher ist, erfährt man dadurch, wie weit die Schraube in den Knochen eingedrungen ist.

Damit die Gewebeschutzhülse die ihr zugedachte Position am Knochen genau einhält, ist ihr vorderes Ende zweckmäßigerweise mit einem Kranz von Zähnen oder dergleichen versehen.

Die Gewebeschutzhülse wird zweckmäßigerweise zusammen mit einem scharfen Mandrin durch die Weichteile an den Knochen herangetrieben, wie dies für ein Trokar bekannt ist. Danach wird der Mandrin entfernt und durch das jeweilige Werkzeug ersetzt.

Die Erfindung wird im folgenden näher unter Bezugnahme auf die Zeichnung erläutert, die ein vorteilhaftes Ausführungsbeispiel veranschaulicht. Es zeigen:
- Fig. 1: den Trokar und
- Fig. 2: ein als Bohrer ausgebildetes Werkzeug.

Der Trokar besteht aus einer Gewebeschutzhülse 1, mit der ein Handgriff 2 starr verbunden ist, und einem Mandrin 3 mit scharfer Spitze. Der Trokar kann in bekannter Weise, mit der Spitze 4 des Mandrins 3 voran, durch das Gewebe zu der gewünschten Knochenposition gestoßen werden. Am vorderen Ende weist die Gewebeschutzhülse 5 einen Kranz von Zähnen 5 auf, die gegen die Knochenoberfläche gedrückt werden, um die Gewebeschutzhülse in der gewählten Position zu halten. Die Auswahl der Position geschieht in der Regel unter Röntgenkontrolle.

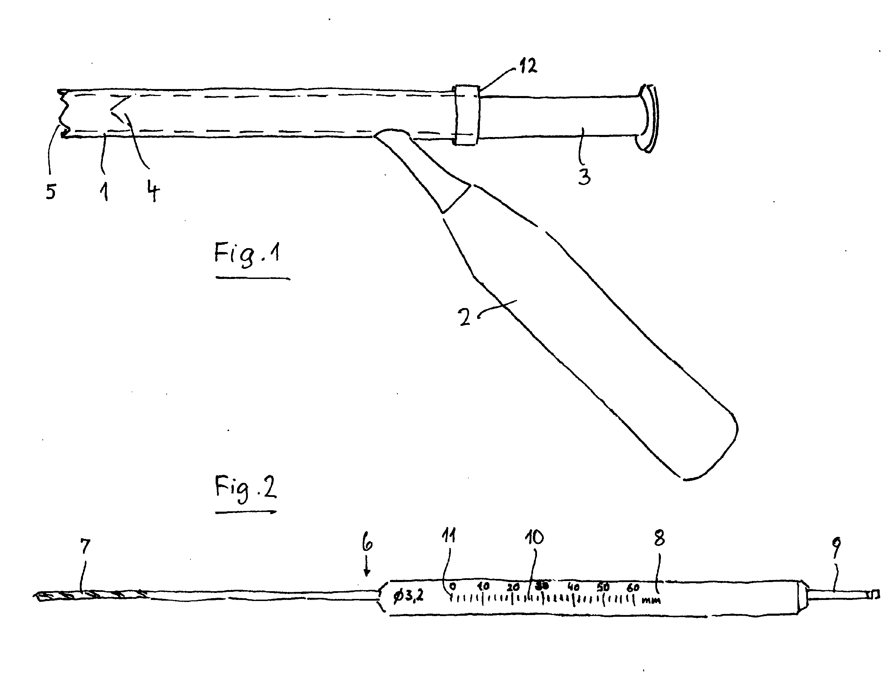

Danach wird der Mandrin 3 entfernt und ein Werkzeug eingesetzt. Es kann sich dabei um den in Fig. 2 dargestellten Bohrer 6 handeln, der aus dem eigentlichen Werkzeugteil 7 und einem Führungsabschnitt 8 sowie gegebenenfalls einem Ansatz 9 zur Verbindung mit einem Griff oder Antrieb besteht. Der Führungsabschnitt 8 hat einen Außendurchmesser, der zu dem Innendurchmesser der Gewebeschutzhülse 1 paßt. Das heißt, daß der Außendurchmesser des Führungsabschnitts 8 einen geringfügig kleineren Durchmesser hat als der Innendurchmesser der Gewebeschutzhülse, so daß einerseits eine gute Führung und andererseits eine leichte Bewegbarkeit des Werkzeugs gewährleistet ist. In jedem Fall ist der Durchmesser des Führungsabschnitts 8 beträchtlich größer als derjenige des eigentlichen Werkzeugs 7. Er wird von den Abmessungen des größten durch die Gewebeschutzhülse einzubringenden Teils bestimmt, beispielsweise durch den Durchmesser des Kopfs einer Knochenschraube.

Das in Fig. 2 dargestellte Werkzeug ist ein Lochbohrer. Statt dessen kann sein Werkzeugteil auch für andere Bearbeitungen ausgebildet sein, beispielsweise als Gewindebohrer oder Schraubendreher.

Auf dem Führungsabschnitt 8 ist ein Längenmaßstab 10 angebracht. Dessen Nullmarke 11 befindet sich zweckmäßigerweise an derjenigen Stelle, die mit dem hinteren Rand 12 der Gewebeschutzhülse 1 übereinstimmt, wenn beide Teile mit ihren Vorderenden auf einer ebenen Fläche aufsitzen. Während des Bohrvorgangs kann der Operateur dann an der Skala ablesen, wie weit das Werkzeug in den Knochen vorgedrungen ist. Dasselbe gilt auch für andere Werkzeuge als Bohrer.

## Patentansprüche

1. Chirurgischer Instrumentensatz, insbesondere für die minimalinvasive Osteosynthese, der eine Gewebeschutzhülse (1) und ein Werkzeug (6) umfaßt, das einen zum Innendurchmesser der Gewebeschutzhülse (1) passenden Führungsabschnitt (8) aufweist, wobei der Durchmesser des Führungsabschnitts (8) größer ist als der Durchmesser des Werkzeugteils (7) des Werkzeugs (6), wobei der Führungsabschnitt (8) des Werkzeugs (6) eine Längenmeßskala (10) aufweist, **dadurch gekennzeichnet, daß** die Nullmarkierung der Längemeßskala (10) mit dem hinteren Ende der Gewebeschutzhülse korrespondiert, wenn die Spitze des Werkzeugs mit dem vorderen Ende der Gewebeschutzhülse abschließt.

2. Instrumentensatz nach Anspruch 1, **dadurch gekennzeichnet, daß** das Werkzeug (6) ein Bohrer ist.

3. Instrumentensatz nach Anspruch 2, **dadurch gekennzeichnet, daß** der Bohrer ein Gewindebohrer ist.

4. Instrumentensatz nach Anspruch 1, **dadurch gekennzeichnet, daß** das Werkzeug (6) ein Schraubendreher ist.

5. Instrumentensatz nach einem der Ansprüche 1 bis 4, **dadurch gekennzeichnet, daß** die Gewebeschutzhülse (1) Teil eines Trokars mit scharfem Mandrin (3) ist.

6. Instrumentensatz nach einem der Ansprüche 1 bis 5, **dadurch gekennzeichnet, daß** das vordere Ende der Gewebeschutzhülse (1) Zähne (5) aufweist.

## Claims

1. Surgical instrument set, in particular for minimally invasive osteosynthesis, comprising a tissue-protecting sleeve (1) and a tool (6), which has a guide section (8) that fits the internal diameter of the tissue-protecting sleeve (1), the diameter of the guide section (8) being greater than the diameter of the tool part (7) of the tool (6), the guide section (8) of the tool (6) having a length-measuring scale (10), **characterized in that** the zero mark of the length-measuring scale (10) corresponds to the rear end of the tissue-protecting sleeve when the tip of the tool is flush with the front end of the tissue-protecting sleeve.

2. Instrument set according to Claim 1, **characterized in that** the tool (6) is a drill.

3. Instrument set according to Claim 2, **characterized in that** the drill is a threaded drill.

4. Instrument set according to Claim 1, **characterized in that** the tool (6) is a screwdriver.

5. Instrument set according to one of Claims 1 to 4, **characterized in that** the tissue-protecting sleeve (1) is part of a trocar with a sharp mandrin (3).

6. Instrument set according to one of Claims 1 to 5, **characterized in that** the front end of the tissue-protecting sleeve (1) has teeth (5).

## Revendications

1. Jeu d'instruments chirurgicaux, en particulier pour l'ostéosynthèse mini-invasive, qui comprend une gaine de protection des tissus (1) et un outil (6) qui présente une portion de guidage (8) s'adaptant au diamètre intérieur de la gaine de protection des tissus (1), le diamètre de la portion de guidage (8) étant supérieur au diamètre de la partie d'outil (7) de l'outil (6), la portion de guidage (8) de l'outil (6) présentant une échelle de mesure de longueur (10), **caractérisé en ce que** le marquage zéro de l'échelle de mesure de longueur (10) correspond à l'extrémité arrière de la gaine de protection des tissus, lorsque la pointe de l'outil se termine avec l'extrémité avant de la gaine de protection des tissus.

2. Jeu d'instruments chirurgicaux selon la revendication 1, **caractérisé en ce que** l'outil (6) est un foret.

3. Jeu d'instruments chirurgicaux selon la revendication 2, **caractérisé en ce que** le foret est un taraud.

4. Jeu d'instruments chirurgicaux selon la revendication 1, **caractérisé en ce que** l'outil (6) est un tournevis.

5. Jeu d'instruments chirurgicaux selon l'une quelconque des revendications 1 à 4, **caractérisé en ce que** la gaine de protection des tissus (1) fait partie d'un trocart avec un mandrin acéré (3).

6. Jeu d'instruments chirurgicaux selon l'une quelconque des revendications 1 à 5, **caractérisé en ce que** l'extrémité avant de la gaine de protection des tissus (1) présente des dents (5).
